(19)

Europäisches Patentamt
European Patent Office
Office européen des brevets

(11) **EP 4 155 767 A1**

(12) **EUROPEAN PATENT APPLICATION**

(43) Date of publication:
**29.03.2023 Bulletin 2023/13**

(21) Application number: **21315189.7**

(22) Date of filing: **24.09.2021**

(51) International Patent Classification (IPC):
**G01S 7/52** $^{(2006.01)}$ **G01S 15/89** $^{(2006.01)}$
**G10K 11/34** $^{(2006.01)}$

(52) Cooperative Patent Classification (CPC):
**G01S 15/8915; G01S 7/52034; G01S 7/5208;
G01S 15/8977; G10K 11/346**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **SuperSonic Imagine
13857 Aix-en-Provence Cedex 3 (FR)**

(72) Inventor: **Fraschini, Christophe
13090 Aix en Provence (FR)**

(74) Representative: **Paustian & Partner Patentanwälte
mbB
Oberanger 32
80331 München (DE)**

(54) **METHOD AND SYSTEM FOR PROCESSING A SET OF SIGNALS RECEIVED BY A TRANSDUCER ELEMENT**

(57)    The invention relates to a method for processing a set of signals of a transducer device comprising a respective set of transducer elements, the method comprising the following steps:

a processing step in which the received set of signals is processed to a plurality of synthetic waves, and
an output step in which the plurality of synthetic waves is outputted through a plurality of channels.

Fig. 1

**Description**

FIELD OF THE DISCLOSURE

The present disclosure relates to methods and systems for processing a set of signals received by a transducer device comprising a respective set of transducer elements. In particular, the method is suitable for providing image data of a medium scanned by the transducer device. For example, the method may be used in a medical device like for instance an ultrasound system.

BACKGROUND OF THE DISCLOSURE

It is known to use a plurality of transducer elements or transceivers (for example arranged as an array) for communication, imaging or scanning purposes, for example in the field of medical imaging, radar, sonar, seismology, wireless communications, radio astronomy, acoustics and biomedicine. One example comprises ultrasound imaging.

In a conventional ultrasound imaging method, an ultrasound transducer device (also referred to as an ultrasound probe) with a set of ultrasound transducer elements may be used. In the method one or several ultrasound pulses are transmitted into a medium by the transducer element in a corresponding transmission step. Then, in a reception step a set of echo signals is received from the medium by the set of transducer elements. In particular, each of the transducer elements converts a received echo signal into for example an electrical signal. The signal may further be processed by the transducer elements. For example, they may be digitalized and/or a signal conditioning step may be carried out.

Conventionally, said signals are then transmitted to a centralized image processing system, wherein each transducer element uses its own channel. The centralized imaging method may further process the received signals to generate an image of the scanned medium, for example using a beamforming method.

This technique has the disadvantage that an increased (data) bandwidth is required between the transducer elements and the centralized image processing system. For example, in case the transducer device comprises 256 transducer elements and 1024 samples are acquired per acoustic firing for imaging the medium, app. 262K data samples per acoustic firing are generated and to be transmitted from the transducer elements the centralized image processing system. Assuming 256 acoustic firing per image, 2 bytes per acquired data sample, and a 80Hz imaging frame rate, a conventional data link between the transducer elements the centralized image processing system requires a data bandwidth of more than 10GB/s.

For this reason, it is for example difficult to implement the data link as a wireless communication, as most conventional wireless communication techniques do not achieve the necessary data bandwidth, in particular not in a reliable manner. Hence, in case a wireless transmission is anyway desired, the data are typically compressed before transmission using a conventional compression technique and/or the (temporal and/or image) resolution is reduced. This technique however has the undesired effect of a deteriorated quality of the final image data what can make a medical examination more difficult and less reliable.

A method for forming medical ultrasound images is disclosed in for example US 6,551,246. The method relates to a synthetic plane wave imaging method. It consists in transmitting plane waves of different angles in the medium, beamforming in receive the backscattered signal then combine the different image to re-synthesize to final image. The transmission of a plane wave on the complete array generates a much higher pressure field than in the synthetic aperture approach for which the transmission may be performed with a single element. Moreover, diffraction and attenuation effects during propagation in soft tissues are significantly lower for an ultrasonic plane wave compared to a single element transmission.

A further method for ultrasound imaging is known for example from US2009234230A1. Said method comprises at least the following steps: a) a transmission step in which a plurality of ultrasonic waves are transmitted into an imaged region and a set of raw data is acquired by an array of transducer elements in response to each ultrasonic wave, the ultrasonic waves having different spatial frequency content; b) a coherence enhancing step in which, for each of a plurality of virtual transmit focal zones in the imaged region, at least one set of coherent data is synthesized from the sets of raw data; c) a beamforming step in which, for each of a plurality of locations included in each of the virtual transmit focal zones, an image pixel is computed by beamforming, using the set of coherent data.

Moreover, Montaldo et.al. proposes to improve the beamforming process by using a coherent recombination of compounded plane-wave transmissions to recover high-quality echographic images without degrading high frame rate capabilities of an ultrasound transducer elements, cf. Montaldo, Gabriel & Tanter, Mickaël & Bercoff, Jeremy & Benech, Nicolás & Fink, Mathias. (2009). Coherent Plane-Wave Compounding for Very High Frame Rate Ultrasonography and Transient Elastography. IEEE transactions on ultrasonics, ferroelectrics, and frequency control. 56. 489-506. 10.1109/TUFFC.2009.1067.

However, these known methods first of all concern the transmission of ultrasound pulses into the medium and do not address the above-mentioned problems.

SUMMARY OF THE DISCLOSURE

Currently, it remains desirable to overcome the aforementioned problems and in particular to provide a method and system for processing a set of signals received by a transducer elements, such that the required data bandwidth and/or the number of required transmission channels for transmitting the processed signals to an external system can be reduced without substantially deteriorating the data quality. In particular, in case the processed signals are outputted to an external processing system configured for image formation based on the received signals, it is desirable that the image quality is not substantially deteriorated due to the data bandwidth reduction and the resulting reduction of the data rate of the outputted data (i.e. the processed signals).

Therefore, according to the embodiments of the present disclosure relates to a method for processing a set of signals received by a transducer elements comprising a respective set of transducer elements. The method comprises the following steps:

- a processing step in which the received set of signals is processed to a plurality of synthetic waves, and
- an output step in which the plurality of synthetic waves is outputted through a plurality of channels.

By providing such a method, it becomes possible to reduce the required data bandwidth for outputting the processed signals, i.e. the plurality of synthetic waves. In other words, the data rate of outputted data can be reduced. This applies in particular to a comparison with a conventional technique, where each transducer element signal may be outputted on an own channel, i.e. where the number of channels correspond to the number of transducer elements.

Accordingly, the output step may be understood as an optimized output step in comparison to the conventional technique.

Furthermore, since the received signals are processed (and/or transformed) to a plurality of synthetic waves, the output data rate of the transducer device and thus the required data bandwidth of a transmission link between the transducer device and an external device (for example the centralized image processing system) can be reduced without substantially loosing information from the original set of signals received by the transducer elements.

As a consequence, it becomes possible to simplify the architecture of the system used to carry out the method, in particular of the output interface of the transducer device.

The reduced data rate also enables a higher acquisition frame rate of the transducer device.

In addition, or alternatively, due to the reduced data rate it becomes possible to use a wireless interface to output the synthetic waves.

Moreover, the computational power required for transmitting and/or processing the synthetic waves may be reduced due to the reduced data rate.

The set of signals received by the transducer elements may be understood as a set of raw data and/or conventional RF data acquired by the transducer elements in response to ultrasonic waves passing the medium. The ultrasonic waves may have for example different spatial frequency content.

The synthetic waves may also be understood as virtual and/or simulated waves, which for instance represent the information contained in the original set of signals.

More in particular, the original set of signals may be approximated with synthetic waves. For example, in an external device the synthetic waves may be beamformed instead of conventional RF data to ensure dynamic receive focusing.

The number of channels may be less than the number of transducer elements of the set of transducer elements. Accordingly, it becomes possible to reduce the required data bandwidth to output the synthetic waves compared to a conventional technique.

For instance, the number of channels may correspond to the number of synthetic waves. In case the number of transducer elements is for example 128 and the number of synthetic waves 40, the data rate can be reduced by a factor of >3.

The received set of signals may be analogue signals and/or the plurality of synthetic waves may constitute digital data. Accordingly, for example the processing step may comprise an A/D (analogue to digital) conversion of the received set of signal. Alternatively, the received set of signals may be A/D converted separately (for example already by the transducer elements), wherein the digitalized set of signals may be processed in the processing step.

The data rate of the plurality of synthetic waves may be reduced compared to a data rate of the received set of signals when digitalized. In other words, a data bandwidth required for outputting the plurality of synthetic waves may be lower than a data bandwidth required for outputting a digitalized set of signals.

The plurality of synthetic waves may be or may comprise at least one of:

- a plurality of diverging waves,
- a plurality of plane waves, and

- a plurality of waves of different phases.

For example, the synthetic waves may be plane waves with different phases, i.e. different reception angles with respect to the transducer elements.

The received set of signals may be a set of backscattered signals. For example, the transducer elements may receive backscattered signals from the medium and transform them in for example electrical signals.

The method may further comprise before the processing step:

- a transmission step in which at least one pulse is transmitted into a medium, and
- a reception step in which a set of echo signals is received from the medium by the set of transducer elements, the set of echo signals being the set of received signals.

For example, the transmission step may comprise insonification of the medium with a cylindrical wave that focuses on a given point and/or a synthetic plane waves of different angles. In the reception step the backscattered echoes of this insonification may be used.

More in particular, in the transmission step a plurality of ultrasonic waves may be transmitted into an imaged region and in the reception step a set of raw data may be acquired by a set of transducer elements in response to each ultrasonic wave, the ultrasonic waves having different spatial frequency content.

The received set of signals may be a set of ultrasound signals.

The transducer device may be an ultrasound transducer device, the transducer elements may be for example in the form of a transducer array, for example a 1D transducer array (having a line of transducer elements), 1.5D transducer array (having one or two lines of transmission transducer elements and one or two lines of reception transducer elements) or 2D or a matrix transducer array (having multiple lines of transducer elements). However, the transducer device is not limited to a transducer element of an ultrasound system. Instead, the transducer device may emit and receive any kind of waves. Further examples comprise a transducer device of a radar system, a sonar system, a seismology system, a wireless communications system, a radio astronomy system, an acoustics system, a Non Destructive Testing (NDT) system, and/or a biomedicine system or the like.

The processing step may comprise a beamforming step in which the received set of signals is beamformed to obtain the plurality of synthetic waves.

The method may further comprise after the output step the following step: an image formation step in which an echographic image is formed based on the plurality of outputted synthetic waves.

Accordingly, the outputted signals may be processed by for example an external device, such as an external processing system, to obtain image data of the medium.

The image formation step may comprise a focused beamforming process based on the plurality of outputted synthetic waves.

The image formation step may comprise forming the echographic image as a function of the geometry of the synthetic waves (or a predefined characteristic of the geometry of the synthetic waves, for example the angles, i.e. phases of the synthetic plane waves).

In other words, the image formation process may take into account the geometry of the synthetic waves, in particular the angles of the synthetic plane waves. Accordingly, a propagation delay of a received signal caused by a angle of a synthetic plane wave may be compensated by taking into account said angle in the image formation step. As a consequence, the data and/or image quality (for example of a formed image) is advantageously not deteriorated by such propagation delays.

The plurality of channels may be physical channels and/or multiplexed (virtual) channels.

The present disclosure further relates to a computer program comprising computer-readable instructions which, when executed by a data processing unit, cause the data processing unit to carry out the method according to the present disclosure. The transducer elements may for example be associated with the data processing unit. The data processing unit may also be referred to as a pre-processing unit.

The present disclosure may also relate to a recording medium readable by a computer and having recorded thereon a computer program including instructions for executing the steps of the method according to the present disclosure, when said program is executed by a computer.

The present disclosure further relates to system for processing a set of signals received by a transducer device comprising a respective set of transducer elements, the system comprising a pre-processing unit configured to:

- process the received set of signals to a plurality of synthetic waves, and
- output the plurality of synthetic waves through a plurality of channels.

The pre-processing unit may be configured to output the plurality of synthetic waves via the plurality of channels to an external (and/or central) processing system, for example be connectable via the plurality of channels to the processing system.

The external processing system may be configured to perform an image formation step in which an echographic image is formed based on the plurality of outputted synthetic waves.

The pre-processing unit may be separate and/or remote to the main processing unit.

The system may comprise the transducer elements.

The system may comprise a probe (for example an ultrasound probe) which may comprise the pre-processing unit and the transducer elements.

The system may be an ultrasound system.

The probe may be configured to output the plurality of synthetic waves to an external processing system via a cable or a wireless communication interface.

The present disclosure may further relate to a platform, for example a medical platform comprising the system according to the disclosure and the external processing system.

The system and/or the platform may comprise further functional characteristics and/or may be configured in correspondence to the method steps described above.

The disclosure and its embodiments may be used in the context of medical systems dedicated to human beings, plants or animals but also any (non-living) soft material to be considered.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, are provided for illustration purposes, and illustrate embodiments of the disclosure and together with the description and serve to support and illustrate the principles thereof.

BRIEF DESCRIPTION OF THE DRAWINGS

Fig. 1 shows an exemplary embodiment of the method according to embodiments of the present disclosure;

Fig. 2 schematically shows an exemplary transducer elements array and an incoming plane wave according to embodiments of the present disclosure;

Fig. 3a schematically shows a spherical wave which is backscattered from a single target reflector in the medium;

Fig. 3b schematically shows the principle of using synthetic plane waves for approximating a backscattered wave according to embodiments of the present disclosure;

Fig. 4a schematically shows a conventional RF data matrix of backscattered signals of a single target reflector;

Fig. 4b schematically shows a plane waves RF data matrix according to embodiments of the present disclosure;

Fig. 5a schematically shows an exemplary reception scheme of a conventional image formation process using signals received by a transducer element array;

Fig. 5b schematically shows an exemplary reception scheme of an image formation process according to embodiments of the present disclosure, and

Fig. 6 shows an exemplary embodiment of a system according to embodiments of the present disclosure, in particular of an ultrasound platform according to embodiments of the present disclosure.

DESCRIPTION OF THE EMBODIMENTS

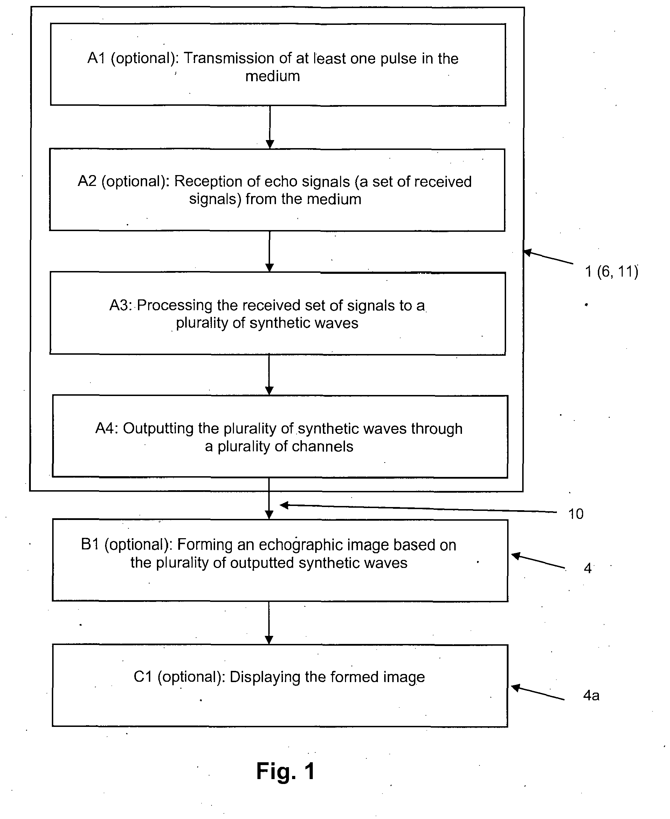

Reference will now be made in detail to exemplary embodiments of the disclosure, examples of which are illustrated in the accompanying drawings. Wherever possible, the same reference numbers will be used throughout the drawings to refer to the same or like parts. Moreover, the features explained in context of a specific embodiment, for example that one of fig. 1, also apply to any one of the other embodiments, when appropriate, unless differently described.

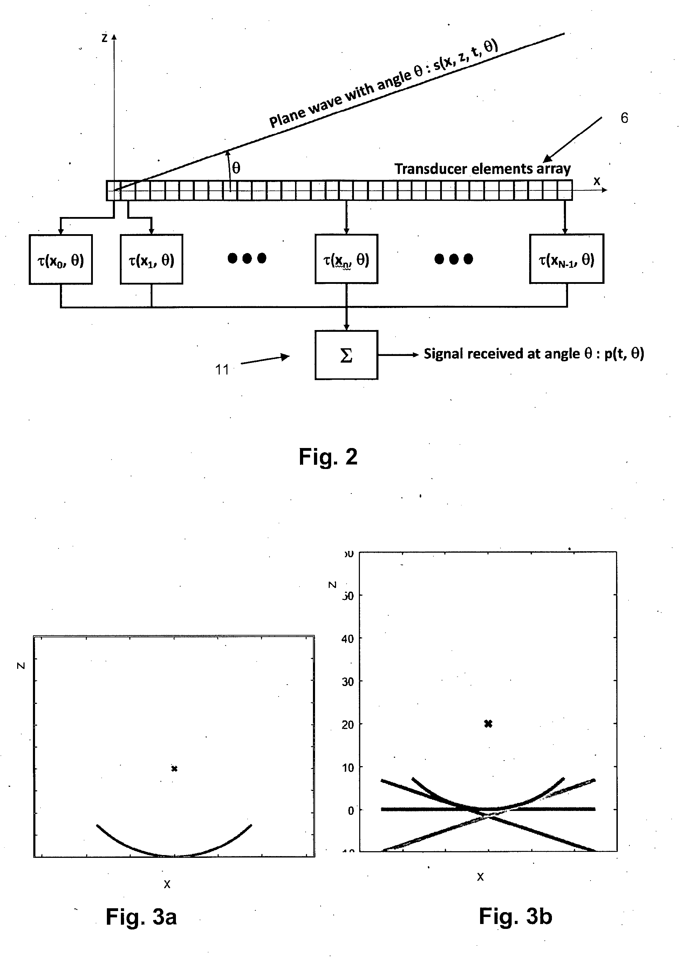

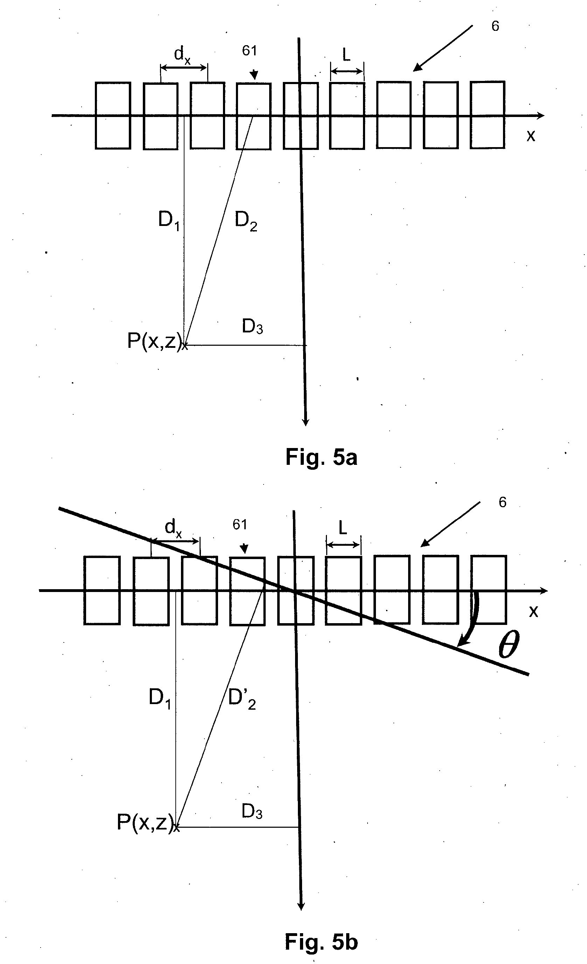

Fig. 1 shows an exemplary embodiment of the method according to embodiments of the present disclosure. The method may be carried out by means of a system 1, more in particular by an ultrasound platform 20. Examples are described in context of fig. 2 and 5.

The method may be an ultrasound method carried out by an ultrasound system. Possible ultrasound methods comprise B-mode imaging, shear wave elastography imaging (such as ShearWave® mode developed by the applicant, ultrafast™ Doppler imaging or angio mode named under Angio P.L.U.S ™ultrasound imaging or any other ultrasound imaging mode. However, the method according to the present disclosure may also be applied to other technical fields than ultrasound examination. In particular, any technical field is possible which use a plurality of transducer elements to acquire data/signals of an examined medium or environment and/or which may optionally use a beamforming technique based on the collected data/signals. Examples comprise methods using a radar system, sonar system, seismology system, wireless communications system, radio astronomy system, acoustics system, Non Destructive Testing (NDT) system and biomedicine system. The principle of acquiring data by a plurality of transducer elements, what would conventionally lead to a corresponding number of channels, is always similar. Accordingly, the method according to the present disclosure may in each of these cases achieve the same positive technical effects as described above, for example of a data reduction. However, for mere illustration purposes of the present disclosure, in the following it is referred to the example of an ultrasound method.

Steps A1 to A4 may be carried out by the system 1 according to the present disclosure, more for example by an ultrasound probe 1. The system is desirably a hand-held system. For example, the system may comprise a transducer elements 6 which may carry out steps A1 and A2 and a pre-processing unit 11 which may carry out steps A3 and A4. The output of the system 1 may be transmitted via an interface 10 to a central or main or external processing system 4.

Step B1 may be carried out by a central (or main or external) processing system 4. Step C1 may be carried out by a display or screen 4a associated with the processing system 4. However, step B1 may also be carried out by the system 1. For example, the image data generated in B1 may then be transmitted from the system 1 to a display 4a (for example wirelessly). It is also possible that the system 1 comprises a display and carries out all steps A1 to C1.

Steps A1 to C1 may be carried out successively, i.e. one after another. However, steps A1 and A2 may be carried out several times before step A3 is carried out based on the collected echo signals of the several steps A2. For example, reception step A2 may be carried out several times (for example 512 times) for each transmission step A1. Moreover, the system 1 may first collet the data of these several steps A2 before outputting them together, and/or the system 4 may also buffer these outputs before steps B1 and B3 are carried out. In addition, the method of fig. 1 may be carried out repeatedly.

The method may comprise the following steps:

In an optional step A1 at least one pulse is transmitted into a medium. For example, the transmission step may comprise insonification of the medium with a cylindrical wave that focuses on a given point and/or plane waves of different angles. More in particular, in the transmission step a plurality of ultrasonic waves may be transmitted into an imaged region.

In an optional step A2 a set of echo signals is received from the medium by the set of transducer elements, the set of echo signals being the set of received signals. In the reception step the backscattered echoes of the insonification of step A1 may be used. More in particular, in the reception step a set of raw data may be acquired by a set of transducer elements in response to each ultrasonic wave. The received set of signals may be a set of ultrasound signals.

In a step A3 the received set of signals is processed to a plurality of synthetic waves. For example, the processing step may comprise a (pre-)beamforming step in which the received set of signals is beamformed to obtain the plurality of synthetic waves, an exemplary embodiment is described below in context of fig. 2. The pre-beamforming step may be performed prior to analogic to digital conversion, i.e. using an analogic processor, or it may be performed after analogic to digital conversion, thus using a digital processor.

In a step A4 the plurality of synthetic waves is outputted through a plurality of channels, for example via the interface 10 to an external system 4.

In an optional step B1 an echographic image is formed based on the plurality of outputted synthetic waves. This image formation step may comprise a focused beamforming process based on the plurality of outputted synthetic waves. The beamforming process may take into account the geometry of the synthetic waves, for example the angles of synthetic plane waves.

In an optional step C1 the formed image is displayed on an electronic display or a screen.

Fig. 2 schematically shows an exemplary transducer elements array and a plane wave according to embodiments of the present disclosure.

The transducer elements 6 may be arranged in the form of a transducer element array along an x-axis with transducer elements $X_0$ to $X_{N-1}$. The received signals $\tau(X_{0 - N-1}, \theta)$ representing ultrasound waves are collected and processed together by the pre-processing unit 11 to obtain a plurality of synthetic plane waves with different phases or angles $\theta$.

Generally, the expression of a plane wave in a specific plane may have the form: s(x, z, t, $\theta$), where x is the coordinate along which the transducer elements are arranges, z is the coordinate representing the depth direction of the medium, and t is the time. A plane wave may be determined by:

$$s(x, z, t, \theta) = A e^{i(\omega t - kx\sin\theta - kz\cos\theta)} \qquad (1)$$

taking into account:

$$\omega = 2\pi f, \; k = \frac{2\pi}{\lambda}, \; \lambda = \frac{c}{f} \qquad (2, 3, 4)$$

where A is a predefined amplitude which may be set as a function of the power of the received ultrasound signals, c is the wave speed and f is the central wave frequency.

The synthetic plane waves processed and output by the system 1 may have the form: p(t, θ). The processing (i.e. calculation steps) are explained in the following with reference to a Fourier transform.

Generally, a Fourier transform can be described by:

$$s(t, x, z) \;\overset{FT}{\leftrightarrow}\; S(f_t, f_x, f_z) \qquad (3)$$

$$S(f_t, f_x, f_z) = \iiint_{-\infty}^{+\infty} s(t, x, z)e^{-2i\pi(f_t t + f_x x + f_z z)}dtdxdz \qquad (4)$$

$$s(t, x, z) = \iiint_{-\infty}^{+\infty} S(f_t, f_x, f_z)e^{2i\pi(f_t t + f_x x + f_z z)}df_t df_x df_z \qquad (5)$$

The Fourier transform can be seen as an expansion of a function s(t,x,z) into a linear combination of elementary functions of the form $e^{2i\pi(f_t t + f_x x + f_z z)}$.

Now, with reference to the present disclosure, in particular with reference to the synthetic waves to be processed by the method according to the present disclosure, $e^{2i\pi(f_t t + f_x x + f_z z)}$ can be considered as a plane wave with temporal frequency $f_t$ and angle $\theta$:

$$\theta = atan\left(\frac{f_x}{f_z}\right) \qquad (6)$$

Accordingly, the Fourier transform can be seen as an expansion of a function s(t,x,z) into a linear combination of plane waves.

Taking discrete version of eq. (5) yields to:

$$s(n_t, n_x, n_z) = \sum_{m_t=0}^{M_t-1} \sum_{m_x=0}^{M_x-1} \sum_{m_z=0}^{M_z-1} S(m_t, m_x, m_z)e^{2i\pi\left(\frac{m_t n_t}{M_t} + \frac{m_x n_x}{M_x} + \frac{m_z n_z}{M_z}\right)} \qquad (7)$$

with the following approximation:

$$s(n_t, n_x, n_z) \cong \sum_{m_t=0}^{M_t-1} \sum_{m_x=0}^{P} \sum_{m_z=0}^{Q} S(m_t, m_x, m_z)e^{2i\pi\left(\frac{m_t n_t}{M_t} + \frac{m_x n_x}{M_x} + \frac{m_z n_z}{M_z}\right)} \qquad (8)$$

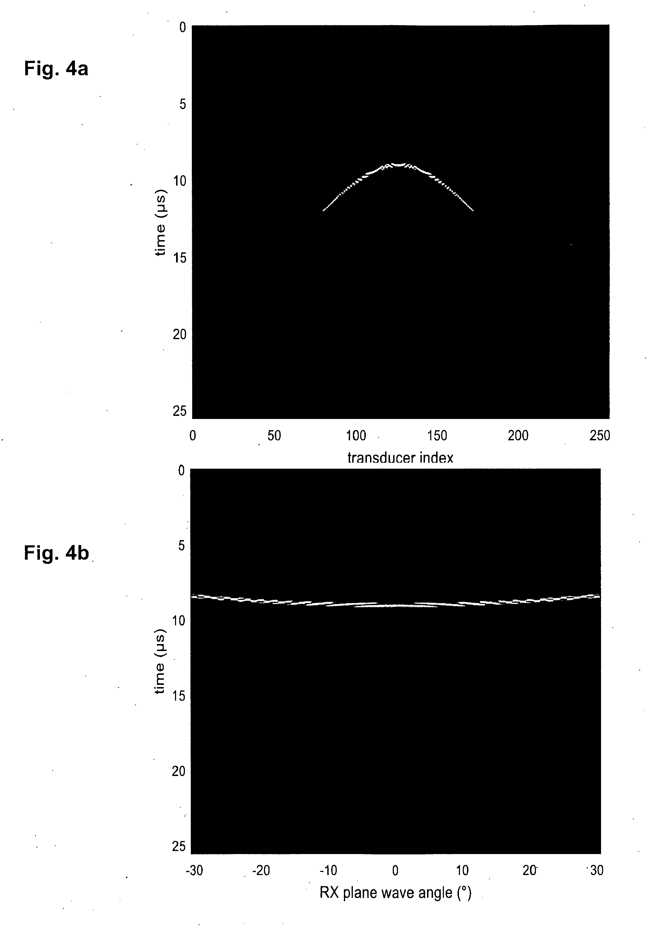

with $P < M_x$, $Q < M_z$, where P*Q is the number of used synthetic waves, and t is a discretized time value, x is a discretized x-axis value, and z is a discretized z-axis value, Mt is the number of samples (in the example of fig. 4b $M_t$ is 512), and $M_x$, $M_z$ are predefined selectable values.

Consequently, as far as the Fourier transform of a wave (function of time and space) exists, it can be expanded (or approximated) into (by) plane waves series. The error depends on P and Q with respect to the frequencies content of the wave.

Fig. 3a schematically shows a spherical wave which is backscattered from a single target reflector in the medium (indicated by a cross). Correspondingly, in a conventional method a dynamic receive focusing may be performed to obtain an ultrasound image. However, in the present disclosure another technique is illustrated as shown for example in fig. 3b.

Fig. 3b schematically shows the principle of using synthetic plane waves for approximating a backscattered wave according to embodiments of the present disclosure. As shown, the plane waves having different angles can approximate the backscattered spherical (and diverging) wave of fig. 3a and hence the conventional dynamic receive focusing. The synthetic plane waves may be obtained in a pre-beamforming step in the system 1 before being outputted to an external system 4. The plane waves may then be beamformed, for example in the system 4, to obtain a dynamic receive focusing.

For example, around 40 plane waves allow to obtain an equivalent final image (in view of image resolution and/or quality) as focused that would conventionally requires around 120 acquisition channels provided by a respective number of transducer elements (for example 128 transducer elements or more). Hence, the proposed method can overcome the framerate lowering caused by propagation time between the system 1 (being for example a probe 1) and the external system 4. This allows hence a decrease of the amount of RF data to be transferred from the system 1 to system 4 by a factor > 3. The method further allows an optimised trade-off between image quality and framerate. Moreover, the method works both on conventional imaging system (focused transmit) and synthetic aperture-based systems, for example B mode, color Doppler, shear wave elastography, ultrafast Doppler or Angio Plus.

The theory of wave expansion described above can be easily extended to 3 dimensions for matrix array transducer elements. In that case, the factor of data size reduction is elevated to the square (factor 3 becomes 9).

Fig. 4a schematically shows a conventional RF data matrix of backscattered signals of a single target reflector with a transducer index on its horizontal (here "x-") axis and time (in $\mu$s) on its vertical axis. In the shown example, a transducer elements with 256 transducer elements is used and 512 samples are collected. Consequently, 256 elements x 512 samples = 131072 samples are collected per acoustic firing. Said raw RF data would conventionally be outputted by a conventional probe and be transmitted to an external system. Assuming 128 acoustic firings per image, 2 bytes per acquired data sample, and an 80Hz imaging frame rate, the correspondingly required data bandwidth would hence be more than 5GB/s.

Fig. 4b schematically shows a plane waves RF data matrix according to embodiments of the present disclosure indicating a RX plane wave angle (°) (i.e. phase) on its horizontal axis and time (in $\mu$s) on its vertical axis. The synthetic plane waves may be obtained based on a respective transformation of the original RF data collected by the transducer (for example as shown in fig. 4a). The slightly bent shape of the exemplary RF data matrix shown in fig. 4b may be due to time delays caused by the angles of the synthetic plane waves. Such angle dependent propagation delays may be compensated in an image formation process according to embodiments of the present disclosure, as described in the following in context of fig. 5b .

In the shown example, 60 plane waves are used and 512 samples are collected. Consequently, 60 plane waves x 512 samples = 30720 samples are collected per acoustic firing. Said raw RF date would conventionally be outputted by a conventional probe and be transmitted to an external system. The correspondingly required data bandwidth would hence be more less than a fourth compared to the conventional example of fig. 4a.

Fig. 5a schematically shows an exemplary reception scheme of a conventional image formation process using signals received by a transducer element array. Fig. 5b schematically shows an exemplary reception scheme of an image formation process according to embodiments of the present disclosure. In both fig. 5a and 5b the transducer elements 6 may be arranged in the form of a transducer element array along an x-axis (i.e. the horizontal axis in fig. 5a and 5b). For example, the transducer element array may correspond to that one described in context of fig. 2. The width of a single transducer element may be L. The distance between two adjacent transducer elements may be $d_x$.

The z-axis in fig. 5a and 5b (i.e. the vertical axis) may represent a principal scanning direction of the transducer element array and/or a direction into the interior of a scanned medium. In particular, fig. 5a and 5b may show exemplary reception schemes of a wave backscattered from a point P(x,z) using for example transducer element 61 (and/or any other transducer element). Said point may be at the position $x=-D_3$, $z=D_1$.

In the method according to the present disclosure the signals received by the transducer elements may be collected and processed together to obtain a plurality of synthetic plane waves with different angles or angles. In the exemplary reception scheme of fig. 5b one exemplary synthetic plane wave with an angle$\theta$ is shown.

An echographic image may be formed based on the plurality of synthetic plane waves. This image formation process (or step) may comprise a focused beamforming process based on the plurality of synthetic waves. The image formation process may take into account the geometry of the synthetic waves, in particular the angles of the synthetic plane waves. Accordingly, the reception delay $D_2/c$ of a conventional reception scheme (as shown in fig. 5a) may be replaced by $D'_2/c$ (as shown in fig. 5b), c being the speed of the wave in the medium. The propagation delay $D_1/c$ may

remain unchanged, i.e. the same in the reception schemes of fig. 5a and 5b. It is noted that in fig. 5a and 5b the distances $D_2/D'_2$ are only schematically shown, in order to reflect the influence of angle $\theta$ of the exemplary synthetic plane wave in fig. 5b.

Therefore, in an image formation process the propagation delay may be compensated as a function of the geometry of the synthetic waves, in particular the angles of the synthetic plane waves. As a consequence, the image quality of a formed image is advantageously not deteriorated by the respective propagation delays.

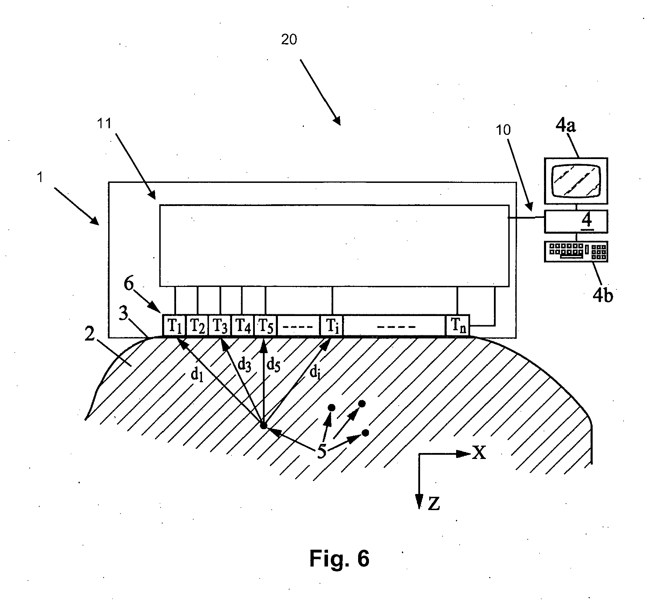

Fig. 6 shows an exemplary embodiment of a system according to embodiments of the present disclosure, in particular of an ultrasound platform according to embodiments of the present disclosure. The platform 20 may comprise the system 1 according to the present disclosure. The system and/or the platform may be configured to carry out the method according to the present disclosure.

The platform 20 shown in fig. 6 may be configured to provide ultrasound images of a viscoelastic medium 2. In response to compressional ultrasound waves the medium 2 scatters. The medium may be for example a human or animal body, for example a part of the body of a patient (breast, liver, abdomen, ...), in the case of medical applications. This system 1 is also configured to monitor the propagation of elastic shear waves to provide images of the elasticity of the medium 2. ,

The images of the medium are produced, for example, by means of a processing system 4 (comprising at least an input interface 4b such as a keyboard or the like, and an output interface 4a such as a screen or the like) or any other electronic central unit, which causes compressional ultrasonic waves to be sent into the medium 2 from its outer surface 3. Said waves interact with scattering particles 5 contained in the medium 2, which particles are reflective for the compressional ultrasonic waves. The particles 5 can be constituted by any heterogeneity of the medium 2, and in particular, when it comes to a medical application, by collagen particles present in human tissues (these particles form on the ultrasound images points known as "speckle").

In order to observe the medium 2 and to generate images of the medium, a system in the form of an ultrasound probe 1 may be used, placed against the outer surface 3 of the observed medium 2. This probe, in particular its transducer elements 6, sends, along a Z axis, compressional ultrasonic wave pulses of the type commonly used in echography, at a frequency of, for example, between 0.5 and 100 MHz and preferably between 0.5 and 15 MHz, for example of the order of 4 MHz.

The transducer elements 6 may consist of an array of n ultrasonic transducer elements T1, T2, ..., Ti, ..., Tn, n being an integer greater than or at least equal to one (1).

This transducer elements 6 may be, for example, in the form of a linear array which can comprise, for example, n = 128 or 256 transducer elements aligned along an X axis perpendicular to the Z axis. The transducer elements in question may also be a two-dimensional array (planar or not) of transducer elements.

The transducer elements T1, T2, ... Tn may be controlled independently of each other by the pre-processing unit 11 and/or the processing system 4. The the pre-processing unit 11 and/or the processing system 4 may comprise at least one central processing unit (CPU) and/or at least one graphic processing unit (GPU).

The transducer elements T1-Tn may thus selectively emit:

- either a "plane" compressional ultrasonic wave (i.e. in this case a wave whose wavefront is rectilinear in the X, Z plane) or any other type of unfocused wave illuminating the entire field of observation in the medium 2, for example a wave generated by having the various transducer elements T1-Tn emit random acoustic signals,
- or a compressional ultrasound wave focused on one or more points of the medium 2.

Optionally a synthetic imaging technique may be applied that uses several unfocused compressional waves, for example plane waves of different angles. Respective echo waves of these plane waves may be combined to obtain in an image of the medium with improved quality.

The system 1 may in particular be in the form of an ultrasound probe which comprises a transducer elements 6 and a pre-processing unit 11. The transducer elements 6 may collect a set of signals and transmit it to the pre-processing unit 11 The pre-processing unit processes the set of signals to a plurality of synthetic waves. The plurality of synthetic waves is outputted by the system, more in particular by the pre-processing unit 11, via a transmission interface 10 (for example a cable or a wireless communication interface) to the processing system 4.

The interface 11 may comprise a multi-channel link (for example physical channels or and/or logical multiplexed channels). The number of channels desirably corresponds to the number of synthetic waves. Due to the data reduction according to the present disclosure, the required data bandwidth of the interface may be for example less than 1 GB/s.

The pre-processing unit may comprise one or several sub-units (not shown in fig. 6), for example at least one of: a transmit-receive switch connected to the transducer elements, a signal conditioning unit, connected to the transmit-receive switch, a RX (i.e. receive) flat processing unit connected to the signal conditioning unit. The RX (i.e. receive) flat processing unit may be configured to process and/or transform the set of signals to a plurality of synthetic waves, as for example described above. The flat processing unit may for example be configured to carry out a beamforming step in which the received set of signals is beamformed to obtain the plurality of synthetic waves. The flat processing unit may hence for example be a receive plane waves (RX flat) beamformer.

Accordingly, a receive plane waves (RX flat) beamformer may be inserted in the conventional architecture of an ultrasound probe/system in between the signal conditioning unit and the bus or interface that connects the probe/system 1 and the processing system 4. Then the receive focused beamformer may be adapted to incoming plane waves to ensure dynamic receive focusing. It has been shown that for example 40 plane waves approximate a diverging or focusing wave. Conventional architectures propose up to for example 256 receive channels corresponding to 256 transducer elements. Using the proposed architecture results in a for example 40 channels (one per synthetic wave) transmission link to connect the system (i.e. the probe) to the processing system 4, that can be a factor 6 in terms of reduction of data to be transferred for the same acquisition. Accordingly, this technique can be used to decrease the capacity of the transmission link 10 or to increase the framerate at a constant channel capacity.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

Although the present disclosure herein has been described with reference to particular embodiments, it is to be understood that these embodiments are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

**Claims**

1. A method for processing a set of signals of a transducer device comprising a respective set of transducer elements, the method comprising the following steps:

   a processing step in which the received set of signals is processed to a plurality of synthetic waves, and
   an output step in which the plurality of synthetic waves is outputted through a plurality of channels.

2. The method according to claim 1, wherein

   the number of channels is less than the number of transducer elements of the set of transducer elements, and/or the number of channels corresponds to the number of synthetic waves, and/or the data ratee of the plurality of synthetic waves is reduced compared to a data rate the received set of signals when digitalized.

3. The method according to claim 1 or 2, wherein
   the plurality of synthetic waves is re-combinable and/or compoundable to approximate a backscattered wave represented by the received set of signals.

4. The method according to any one of the preceding claims, wherein
   the plurality of synthetic waves is at least one of:

   a plurality of diverging waves,
   a plurality of plane waves, and
   a plurality of waves of different phases, and/or
   the received set of signals is a set of backscattered signals.

5. The method according to any one of the preceding claims,
   further comprising the following steps before the processing step:

   a transmission step in which at least one pulse is transmitted into a medium,
   and
   a reception step in which a set of echo signals is received from the medium by the set of transducer elements, the set of echo signals being the set of received signals.

6. The method according to any one of the preceding claims, wherein

the received set of signals is a set of ultrasound signals, and/or
the transducer device is an ultrasound transducer device, and/or
the transducer device is a matrix array transducer device.

7. The method according to any one of the preceding claims, wherein
the processing step comprises a beamforming step in which the received set of signals is beamformed to obtain
the plurality of synthetic waves.

8. The method according to any one of the preceding claims,
further comprising after the output step the following step:
an image formation step in which an echographic image is formed based on the plurality of outputted synthetic waves.

9. The method according to the preceding claim, wherein
the image formation step comprises:

processing a focused beamforming based on the plurality of outputted synthetic waves, and/or
forming the echographic image as a function of the geometry of the synthetic waves.

10. The method according to any one of the preceding claims, wherein
the plurality of channels are physical channels and/or multiplexed channels.

11. A computer program comprising computer-readable instructions which when executed by a data processing unit
cause the data processing unit to carry out the method according to any one of preceding method claims.

12. A system for processing a set of signals received by a transducer device comprising a respective set of transducer
elements, the system comprising a pre-processing unit configured to:

process the received set of signals to a plurality of synthetic waves,
output the plurality of synthetic waves through a plurality of channels.

13. The system according to the preceding claim, wherein

the pre-processing unit is configured to output the plurality of synthetic waves via the plurality of channels to
an external processing system which is configured to perform an image formation step in which an echographic
image is formed based on the plurality of outputted synthetic waves, and/or
the pre-processing unit is separate and/or remote to the main processing unit.

14. The system according to any one of the preceding claims 12 or 14, further comprising a probe which comprises the
pre-processing unit and the transducer device.

15. The system according to any one of the preceding claims 12 to 14, wherein the probe is configured to output the
plurality of synthetic waves to an external processing system via a cable or a wireless communication interface.

EP 4 155 767 A1

A1 (optional): Transmission of at least one pulse in the
medium

A2 (optional): Reception of echo signals (a set of received
signals) from the medium

1 (6, 11)

A3: Processing the received set of signals to a
plurality of synthetic waves

A4: Outputting the plurality of synthetic waves through
a plurality of channels

10

B1 (optional): Forming an echographic image based on
the plurality of outputted synthetic waves

4

C1 (optional): Displaying the formed image

4a

**Fig. 1**

12

**Fig. 2**

**Fig. 3a**

**Fig. 3b**

Fig. 4a

Fig. 4b

**Fig. 5a**

**Fig. 5b**

**Fig. 6**

Europäisches
Patentamt

European
Patent Office

Office européen
des brevets

# EUROPEAN SEARCH REPORT

Application Number

EP 21 31 5189

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | COLAS SCHRETTER ET AL: "Ultrasound Imaging From Sparse RF Samples Using System Point Spread Functions", IEEE TRANSACTIONS ON ULTRASONICS, FERROELECTRICS, AND FREQUENCY CONTROL, vol. 65, no. 3, 1 March 2018 (2018-03-01), pages 316-326, XP055590673, USA ISSN: 0885-3010, DOI: 10.1109/TUFFC.2017.2772916 * the whole document * | 1-15 | INV. G01S7/52 G01S15/89 G10K11/34 |
| X | US 2016/349367 A1 (DUNCAN DAVID P [US]) 1 December 2016 (2016-12-01) * abstract *; figures 1, 2, 4, 5 * * paragraph [0001] - paragraph [0006] * * paragraph [0014] - paragraph [0077] * | 1-15 | |
| A | MOHAMED MANSOUR ET AL: "Lossless compression of ultrasound RF data", ULTRASONICS SYMPOSIUM (IUS), 2010 IEEE, IEEE, 11 October 2010 (2010-10-11), pages 2282-2285, XP031952723, DOI: 10.1109/ULTSYM.2010.5935587 ISBN: 978-1-4577-0382-9 * the whole document * | 1-15 | TECHNICAL FIELDS SEARCHED (IPC) G01S G10K |

The present search report has been drawn up for all claims

| Place of search | Date of completion of the search | Examiner |
|---|---|---|
| Munich | 25 February 2022 | Zaneboni, Thomas |

## ANNEX TO THE EUROPEAN SEARCH REPORT
## ON EUROPEAN PATENT APPLICATION NO.

**EP 21 31 5189**

This annex lists the patent family members relating to the patent documents cited in  the above-mentioned European search report.
The members are as contained in the European Patent Office EDP file on
The European Patent Office is in no way liable for these particulars which are merely given for the purpose of information.

**25-02-2022**

| Patent document cited in search report | | Publication date | Patent family member(s) | | Publication date |
|---|---|---|---|---|---|
| US 2016349367 | A1 | 01-12-2016 | CN | 106210719 A | 07-12-2016 |
| | | | US | 2016349367 A1 | 01-12-2016 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6551246 B

- US 2009234230 A1

**Non-patent literature cited in the description**

- **MONTALDO, GABRIEL ; TANTER, MICKAËL ; BERCOFF, JEREMY ; BENECH, NICOLÁS ; FINK, MATHIAS.** Coherent Plane-Wave Compounding for Very High Frame Rate Ultrasonography and Transient Elastography. *IEEE transactions on ultrasonics, ferroelectrics, and frequency control,* 2009, vol. 56, 489-506